# EUROPEAN PATENT APPLICATION

(11) **EP 4 772 220 A1**
(43) Date of publication of application: **08.07.2026**
(21) Application number: 24876565.3
(22) Date of filing: 10.10.2024
(51) Int. Cl.: A61M 16/00, A61M 16/10, A61M 16/16, A61M 39/26, B08B 17/04, F16L 37/084

(54) **VENTILATOR AIR OUTLET SLIDING PROTECTION DEVICE**

(30) Priority: 10.10.2023 CN 202311308075; 10.10.2023 CN 202322717080 U; 10.10.2023 CN 202311308100; 10.10.2023 CN 202322717084 U
(71) Applicant: COFOE MEDICAL TECHNOLOGY CO., LTD., Changsha, Hunan 410007 (CN)
(72) Inventor: YANG, Quangang, Changsha, Hunan 410007 (CN); ZHOU, Ruopeng, Changsha, Hunan 410007 (CN); ZHANG, Min, Changsha, Hunan 410007 (CN)
(74) Representative: Alpspitz IP
(86) International application number: PCT/CN2024/123804
(87) International publication number: WO 2025/077753

(57) **Abstract**

Disclosed in the present invention is a ventilator air outlet sliding protection device, comprising a ventilator main body, a ventilation hose and a protective cover. The ventilator main body comprises a housing, a fan assembly and an air outlet assembly, wherein the fan assembly is provided with a fan air inlet and a fan air outlet, the air outlet assembly is in communication with the fan air outlet and is configured to be connected to the ventilation hose, the air outlet assembly is located in the housing, and the housing is provided with an opening corresponding to the air outlet assembly. The protective cover is arranged in a manner of sliding relative to the housing, and can be opened and closed. When the protective cover is in a closed state, the protective cover covers the air outlet assembly; and when the protective cover is in an open state, the air outlet assembly is in an exposed state. The device of the present invention is simple to use and convenient to operate, and can effectively protect an air outlet of a ventilator to prevent the air outlet, an internal water tank and other pipelines of the ventilator from being contaminated by external dust, bacteria, etc.

## Description

### FIELD OF THE INVENTION

The present invention relates to a sliding protection device for a ventilator air outlet, belonging to the technical field of household ventilator applications.

### BACKGROUND OF THE INVENTION

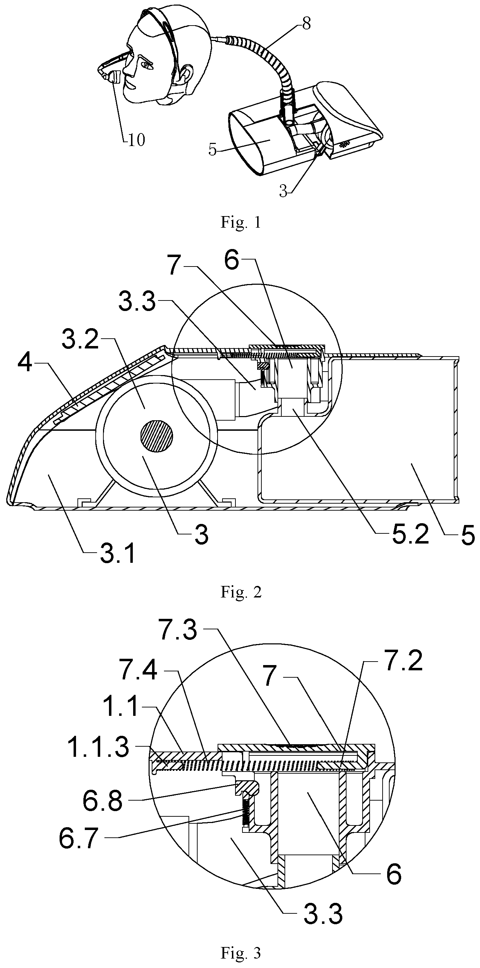

As auxiliary breathing devices, ventilators are increasingly finding their way into families in need. A common ventilator operates as shown in FIG. 1. A turbine fan assembly 3 in the ventilator sucks external air into the ventilator for compression. The compressed air passes through a ventilator water tank 5 and is then blown out through a ventilator air outlet. When in use, a ventilation hose 8 is mounted on the ventilator air outlet and connected to a mask 10. Through the mask 10, a user can inhale the compressed air delivered by the ventilator to assist in breathing.

The existing ventilator air outlet (outlet hose) protrudes outside a ventilator housing, such as a ventilator disclosed in the design patent with the publication number CN306453142S. This design facilitates insertion of the ventilation hose into the air outlet and disengagement of the ventilation hose from the air outlet. However, this design has a drawback: the air outlet is directly exposed to the air without any protective measures. When the ventilator is stored, the air outlet is easily contaminated by dust, bacteria, or other foreign objects in the environment, such as pet hair. When the user uses the ventilator with the contaminated air outlet, the contaminants are delivered by the compressed air into the mask and enter the user's respiratory tract, causing discomfort and even respiratory or lung infections.

### SUMMARY OF THE INVENTION

In order to overcome the problems existing in the prior art, the present invention provides a sliding protection device for a ventilator air outlet, which provides reliable protection for the ventilator air outlet and prevent foreign objects from entering the air outlet. Specific technical solutions are as follows.

A sliding protection device for a ventilator air outlet includes a ventilator main body, a ventilation hose, and a protective cover, where the ventilator main body includes a housing, a fan assembly, and an air outlet assembly; the fan assembly is provided with a fan air inlet and a fan air outlet, the air outlet assembly is in communication with the fan air outlet, and the air outlet assembly is configured to be connected to the ventilation hose, where
the air outlet assembly is located in the housing, and the housing is provided with an opening corresponding to the air outlet assembly; the protective cover is arranged in a manner of sliding relative to the housing, and the protective cover is capable of being opened and closed; when the protective cover is in a closed state, the protective cover covers the air outlet assembly; when the protective cover is in an open state, the air outlet assembly is in an exposed state.

By employing the above technical solution, the conventional design where the air outlet protrudes from the housing is avoided. By designing the air outlet assembly in the housing to cooperate with the sliding protective cover, reliable protection is provided for the air outlet assembly when the ventilator is not in use, and foreign objects are prevented from entering the air outlet assembly. When the ventilator is required to be used, after the protective cover is opened, the ventilation hose is capable of passing through the opening of the housing and be connected to the air outlet assembly.

Furthermore, the air outlet assembly includes a connecting cylinder and a central connecting tube, one end of the connecting cylinder is connected to the housing, another end of the connecting cylinder is connected to a periphery of the central connecting tube, and there is a gap between the connecting cylinder and the central connecting tube; an end of the central connecting tube near the housing does not protrude from the housing. When the protective cover is in the open state, the ventilation hose is inserted through the opening of the housing into the gap between the connecting cylinder and the central connecting tube, and the ventilation hose is in tight fit with the air outlet assembly to fix the ventilation hose.

Furthermore, in response to an insertion operation of connecting the ventilation hose to the air outlet assembly, the protective cover abuts against the ventilation hose. Such design has an advantage: after the ventilation hose is used and the ventilation hose is removed, the protective cover automatically recovers to the closed state under the action of an elastic element.

Furthermore, the sliding protection device further includes a first elastic element and an elastic limiting assembly; and
a fixing seat is provided on the housing, and the protective cover is slidably provided on the fixing seat; when the protective cover is opened, the elastic limiting assembly moves upward to lock the protective cover; when the ventilation hose is inserted into the air outlet assembly, the elastic limiting assembly is driven to move downward and release the locking of the protective cover; when the ventilation hose is removed from the air outlet assembly, the protective cover is closed under the action of the first elastic element.

Furthermore, one end of the first elastic element is connected to the protective cover, and another end of the first elastic element is connected to the housing or the fixing seat. Furthermore, the elastic limiting assembly includes a second elastic element and a limit bracket, where the second elastic element is configured to apply an elastic force to move the limit bracket towards the protective cover; preferably, an elastic force direction of the first elastic element is perpendicular to an elastic force direction of the second elastic element; the limit bracket is slidably arranged on the air outlet assembly, one end of the second elastic element is connected to the limit bracket, and another end of the second elastic element is connected to the air outlet assembly. When the protective cover is in the open state, the ventilation hose is capable of passing through the opening and be connected to the air outlet assembly. When the user inserts the ventilation hose into a ventilation hose fixing assembly, the movement of the limit bracket is actuated to release the locking of the protective cover, and the protective cover abuts against the ventilation hose under the action of the first elastic element. After use and after the ventilation hose is removed, the protective cover automatically recovers to the closed state under the action of the first elastic element. The first elastic element applies an elastic force to recover the protective cover to the closed state. When the ventilator is not in use, the first elastic element ensures that the protective cover is always in the closed state, that is, the protective cover covers the opening of the housing.

Furthermore, the connecting cylinder is provided with a guide hole, and the limit bracket extends through the guide hole. In this way, the limit bracket is reliably and slidably connected to the air outlet assembly.

Furthermore, a channel is provided on a wall of the connecting cylinder, a movable actuator extends through the channel, and an end of the actuator is located in the gap between the connecting cylinder and the central connecting tube.

Furthermore, the actuator is a first oblique wedge, and a second oblique wedge configured to match the first oblique wedge is provided on the limit bracket; when the ventilation hose is inserted into the gap between the connecting cylinder and the central connecting tube, the first oblique wedge is able to slide within the mounting hole. By the oblique wedge, when the ventilation hose is inserted into the gap, the ventilation hose actuates the first oblique wedge to slide horizontally in the opening, so that the second oblique wedge is driven to move the limit bracket to downward. The movement of the limit bracket releases the locking of the protective cover, so that the protective cover abuts against the ventilation hose under the action of the first elastic element. When the ventilation hose is removed, the protective cover automatically recovers to the closed state under the action of the first elastic element,

Furthermore, the limit bracket includes two parallel rods, and the second oblique wedge is connected between the two rods. By such design, the first elastic element is able to pass between the two rods, avoiding interference between the first elastic element and the limit bracket. Meanwhile, the two rods provides better limitation (positioning) for the protective cover, preventing the occurrence of deflection, blockage, etc. in the protective cover due to uneven force distribution.

Furthermore, the channel is a strip-shaped groove extending vertically and passing through the wall of the connecting cylinder, and the actuator is a cross bar that is arranged on the limit bracket and is movable up and down along the strip-shaped groove; when the ventilation hose is inserted into the gap between the connecting cylinder and the central connecting tube, the cross bar is pressed down, and the cross bar drives the limit bracket to move downward. The downward movement of the limit bracket releases the locking of the protective cover, so that the protective cover abuts against the ventilation hose under the action of the first elastic element.

Furthermore, an inner surface of the protective cover is provided with a relief groove corresponding to the limit bracket. The relief groove minimizes mutual interference between the protective cover and the limit bracket during sliding of the protective cover. The inner surface of the protective cover refers to a surface of the protective cover facing the air outlet assembly.

Furthermore, the inner surface of the protective cover is further provided with a positioning groove, and when the protective cover is in the open state, the limit bracket abuts against a side wall of the positioning groove. By designing the positioning groove, the limit bracket does not necessarily abut against an edge of the protective cover, facilitating freer and more flexible arrangement of the limit bracket.

According to another aspect of the present invention, the sliding protection device further includes a first elastic element and a plate that is located in the housing, and the air outlet assembly is connected to the plate; the protective cover is slidably provided between the housing and the plate; a limit element is provided on the plate, and the protective cover is limited by the limit element when opened;
when the ventilation hose is connected to the air outlet assembly, the plate is pressed down, and the plate undergoes elastic deformation, so that the limit element releases the limit on the protective cover; and
after the ventilation hose is disengaged from the air outlet assembly, the protective cover is closed under the action of the first elastic element to cover the air outlet assembly.

When the protective cover is in the open state, the ventilation hose is capable of passing through the opening of the housing and be connected to the air outlet assembly. When the user inserts the ventilation hose into the air outlet assembly, the plate undergoes elastic deformation, so that the limit element moves and disengages from the protective cover, and the protective cover abuts against the ventilation hose under the action of the first elastic element. After use and after the ventilation hose is removed, the protective cover automatically recovers to the closed state under the action of the first elastic element. A space for accommodating the protective cover is formed between the plate and the housing, to limit the sliding range of the protective cover.

Furthermore, one end of the elastic element is connected to the protective cover, and another end of the elastic element is connected to the housing or the plate.

Furthermore, the plate is fixedly connected to a periphery of one end of the connecting cylinder, and a plane where the plate is located is perpendicular to an axial direction of the connecting cylinder; a surface of the plate is provided with the limit element, and the limit element is located on a side of the plate away from the connecting cylinder.

Furthermore, the limit element has an inclined surface on a side near the connecting cylinder.

Furthermore, a hook is provided on the housing, one end of the plate is engaged with the hook, and another end of the plate is fixed to the housing by a fastener. This fixing manner enables rapid fixation of the ventilation hose fixing assembly to the housing, and facilitates accurate positioning of the ventilation hose fixing assembly on the housing. More advantageously, only one end of the plate is fixed by a fastener, and another end of the plate is engaged with the hook. The end of the plate that is engaged with the hook is equivalent to a free end. When the plate is subjected to a pressure perpendicular to the plane where the plate is located, the end of the plate that is engaged with the hook is capable of slight displacement, making the plate more prone to elastic deformation.

According to another aspect of the present invention, furthermore, the ventilator main body further includes a first elastic element and a plate located in the housing; the protective cover is slidably provided between the housing and the plate, one end of the first elastic element is connected to the protective cover, and another end of the first elastic element is connected to the housing or the plate; and the first elastic element is configured to apply an elastic force to switch the protective cover from the open state to the closed state.

Furthermore, the plate is fixedly connected to a periphery of one end of the connecting cylinder near the housing, and a plane where the plate is located is perpendicular to an axial direction of the connecting cylinder; both ends of the plate are provided with positioning protrusions, and the positioning protrusions extend from the plate towards the housing. When the plate is fixed to the housing, an extension height of the positioning protrusion towards the housing is greater than a thickness of the protective cover, thereby ensuring that the protective cover is able to slide in the gap between the housing and the plate.

Furthermore, a hook is provided on the housing, one end of the plate is engaged with the hook, and another end of the plate is fixed to the housing by a fastener. This fixing manner enables rapid fixation of the plate and the air outlet assembly to the housing, and facilitates accurate positioning of the air outlet assembly on the housing.

Furthermore, the protective cover is provided with a guide protrusion, and the plate is provided with a corresponding guide groove matching the guide protrusion; the plate is further provided with a limit buckle used with the guide protrusion, and the limit buckle is located at an end of the guide groove away from the connecting cylinder. When the protective cover slides to switch from the closed state to the open state, the guide protrusion slides within the guide groove until the guide protrusion is engaged in the limit buckle. The limit buckle is used to limit the protective cover and prevent the protective cover from automatically rebounding. A person skilled in the art can understand that the engaging force between the limit protrusion and the limit buckle is capable of preventing the protective cover from rebounding. However, when the user applies an external force to the protective cover, the limit protrusion is capable of disengaging from the limit buckle, and then the protective cover automatically slides under the action of the elastic element to the position where the opening of the housing is closed.

According to another aspect of the present invention, furthermore, a fixing seat is provided on the housing, and the protective cover is slidably provided on the fixing seat; the sliding protection device further includes a first elastic element; one end of the first elastic element is connected to the protective cover, and another end of the first elastic element is connected to the housing or the fixing seat.

Furthermore, the fixing seat is provided with a guide strip and/or a guide groove, and the protective cover is provided with a guide groove and/or a guide strip matching the fixing seat. The guide strip and/or the guide groove ensures that the protective cover is reliably and slidably mounted on the slide cover mounting seat.

Furthermore, the fixing seat is provided with a locking positioning protrusion, and the protective cover is provided with a locking positioning groove matching the locking positioning protrusion. When the protective cover slides to switch from the closed state to the open state, the protective cover slides on the fixing seat until the locking positioning protrusion is engaged in the locking positioning groove. The engagement of the locking positioning protrusion and the locking positioning groove is capable of limiting the protective cover and prevent the protective cover from automatically rebounding under the elastic force of the elastic element. A person skilled in the art can understand that the engaging force between the locking positioning protrusion and the locking positioning groove is capable of preventing the protective cover from rebounding. However, when the user applies an external force to the protective cover, the locking positioning protrusion is capable of disengaging from the locking positioning groove, and then the protective cover automatically slides under the action of the elastic element to the position where the opening of the housing is covered.

Compared with the prior art, the beneficial effects of the present invention are as follows:
The sliding protection device is easy to use and convenient to operate, and effectively protects the ventilator air outlet and prevents the air outlet, an internal water tank and other pipelines of the ventilator from being contaminated by external dust, bacteria, etc. In a preferred solution, in response to the insertion operation of connecting the ventilation hose to the air outlet assembly, the protective cover abuts against the ventilation hose, thereby preventing the user from forgetting to close the protective cover due to original operating habits after using the ventilator, which would otherwise affect the operating effect of the protection device.

### BRIEF DESCRIPTION OF THE DRAWINGS

FIG. 1 is a schematic operating view of an existing ventilator;
FIG. 2 is a middle cross-sectional view of a sliding protection device for a ventilator air outlet in Embodiment 1 of the present invention;
FIG. 3 is an enlarged view of a circled area in FIG. 2;
FIG. 4 is a cross-sectional view of a rod position of a limit bracket of the sliding protection device for the ventilator air outlet in Embodiment 1 of the present invention (with a protective cover in a closed state);
FIG. 5 is an enlarged view of a circled area in FIG. 4;
FIG. 6 is a middle partial cross-sectional view of the sliding protection device for the ventilator air outlet in Embodiment 1 of the present invention (with the protective cover in the closed state);
FIG. 7 is a partial cross-sectional view of the rod position of the limit bracket of the sliding protection device for the ventilator air outlet in Embodiment 1 (or Embodiment 4) of the present invention (with the protective cover in the closed state);
FIG. 8 is a schematic partial view of the protective cover in Embodiment 1 of the present invention in an open state;
FIG. 9 is a schematic view after a ventilation hose is connected to the sliding protection device for the ventilator air outlet in Embodiment 1;
FIG. 10 is an enlarged view of a circled area in FIG. 9:
FIG. 11 is a partial top view of the sliding protection device for the ventilator air outlet in Embodiment 1, with the protective cover removed;
FIG. 12 is a partial bottom view of the sliding protection device for the ventilator air outlet in Embodiment 1, with the protective cover removed;
FIG. 13 is a schematic view (top view) of the protective cover of the sliding protection device for the ventilator air outlet in Embodiment 1;
FIG. 14 is a schematic view (bottom view) of the protective cover of the sliding protection device for the ventilator air outlet in Embodiment 1;
FIG. 15 is a schematic view of the limit bracket of the sliding protection device for the ventilator air outlet in Embodiment 1;
FIG. 16 is a schematic view of a variation of the limit bracket of the sliding protection device for the ventilator air outlet in Embodiment 1;
FIG. 17 is a cross-sectional view of a first oblique wedge of the sliding protection device for the ventilator air outlet in Embodiment 1;
FIG. 18 is a schematic view of a variation of the protective cover of the sliding protection device for the ventilator air outlet in Embodiment 1;
FIG. 19 is a schematic partial view of the protective cover of the sliding protection device for the ventilator air outlet in Embodiment 1 in the open state (another variation of the limit bracket);
FIG. 20 is a middle cross-sectional view of a sliding protection device for a ventilator air outlet in Embodiment 2 (or Embodiment 3) of the present invention;
FIG. 21 is an enlarged view of a circled area in FIG. 20;
FIG. 22 is a middle partial cross-sectional view of the sliding protection device for the ventilator air outlet in Embodiment 2 (or Embodiment 3) of the present invention;
FIG. 23 is a partial cross-sectional view of a screw position of the sliding protection device for the ventilator air outlet in Embodiment 2 (or Embodiment 3) of the present invention (with a protective cover in a closed state);
FIG. 24 is a partial cross-sectional view of a screw position of the sliding protection device for the ventilator air outlet in Embodiment 2 (or Embodiment 3) of the present invention (with the protective cover in an open state);
FIG. 25 is a schematic view of a ventilation hose fixing assembly of the sliding protection device for the ventilator air outlet in Embodiment 2 of the present invention;
FIG. 26 is a cross-sectional view of the ventilation hose fixing assembly of the sliding protection device for the ventilator air outlet in Embodiment 2 of the present invention;
FIG. 27 is an enlarged view of area A in FIG. 25;
FIG. 28 is a schematic view (top view) of the protective cover of the sliding protection device for the ventilator air outlet in Embodiment 2 (or Embodiment 3) of the present invention;
FIG. 29 is a schematic view (bottom view) of the protective cover of the sliding protection device for the ventilator air outlet in Embodiment 2 of the present invention;
FIG. 30 is a schematic view (bottom view) of a variation of the protective cover of the sliding protection device for the ventilator air outlet in Embodiment 2 of the present invention;
FIG. 31 is a partial bottom view of the sliding protection device for the ventilator air outlet in Embodiment 2 (or Embodiment 3) of the present invention (with the protective cover in the closed state);
FIG. 32 is a partial bottom view of the sliding protection device for the ventilator air outlet in Embodiment 2 (or Embodiment 3) of the present invention (with the protective cover in the open state);
FIG. 33 is a bottom view of an upper cover of the sliding protection device for the ventilator air outlet in Embodiment 2 (or Embodiment 3) of the present invention;
FIG. 34 is a schematic view of a variation of the ventilation hose fixing assembly of the sliding protection device for the ventilator air outlet in Embodiment 2 of the present invention;
FIG. 35 is a cross-sectional view of a variation of the ventilation hose fixing assembly of the sliding protection device for the ventilator air outlet in Embodiment 2 of the present invention;
FIG. 36 is a schematic view the protective cover abutting a limit element of the sliding protection device for the ventilator air outlet in Embodiment 2 of the present invention;
FIG. 37 is a schematic view of abutment between the protective cover and a ventilation hose of the sliding protection device for the ventilator air outlet in Embodiment 2 of the present invention;
FIG. 38 is a schematic view of a ventilation hose fixing assembly of a sliding protection device for a ventilator air outlet in Embodiment 3 of the present invention;
FIG. 39 is a cross-sectional view of the ventilation hose fixing assembly of the sliding protection device for the ventilator air outlet in Embodiment 3 of the present invention;
FIG. 40 is a schematic view of the ventilation hose fixing assembly of the sliding protection device for the ventilator air outlet in a variation of Embodiment 3 of the present invention;
FIG. 41 is a partial cross-sectional view of the ventilation hose fixing assembly of the sliding protection device for the ventilator air outlet in a variation of Embodiment 3 of the present invention;
FIG. 42 is a partial enlarged view of the sliding protection device for the ventilator air outlet in a variation of Embodiment 3 of the present invention (with a protective cover in an open state);
FIG. 43 is a middle cross-sectional view of a sliding protection device for a ventilator air outlet in Embodiment 4 of the present invention;
FIG. 44 is an enlarged view of a circled area in FIG. 43;
FIG. 45 is a middle partial cross-sectional view of the sliding protection device for the ventilator air outlet in Embodiment 4 of the present invention (with a protective cover in a closed state);
FIG. 46 is a middle partial cross-sectional view of the sliding protection device for the ventilator air outlet in Embodiment 4 of the present invention (with the protective cover in an open state);
FIG. 47 is a schematic view after a ventilation hose is connected to the sliding protection device for the ventilator air outlet in Embodiment 4 of the present invention;
FIG. 48 is a schematic view (bottom view) of the protective cover of the sliding protection device for the ventilator air outlet in Embodiment 4 of the present invention;
FIG. 49 is a top view of an upper cover of the sliding protection device for the ventilator air outlet in Embodiment 4 of the present invention;
FIG. 50 is a bottom view of the upper cover of the sliding protection device for the ventilator air outlet in Embodiment 4 of the present invention;
FIG. 51 is a schematic view after the ventilation hose is connected to the sliding protection device for the ventilator air outlet in a variation of Embodiment 4 of the present invention;
FIG. 52 is an enlarged view of a circled portion in FIG. 50;
FIG. 53 is a partial schematic view of the sliding protection device for the ventilator air outlet in a variation of Embodiment 4 of the present invention, with the protective cover removed; and
FIG. 54 is a bottom view of the protective cover of the sliding protection device for the ventilator air outlet in a variation of Embodiment 4 of the present invention.

In the figures: 1 - upper cover, 1.1 - fixing seat, 1.1.1 - opening, 1.1.2 - guide groove, 1.1.3 - first guide column, 1.1.4 - locking positioning protrusion, 1.2 - hook, 1.3 - screw post, 1.4 - guide strip, 1.5 - upper cover fixing column, 2 - lower cover, 3 - fan assembly, 3.1 - fan air inlet, 3.2 - fan body, 3.3 - fan air outlet, 4 - control panel, 5 - water tank, 5.1 - water tank air inlet, 5.2 - water tank air outlet, 6 -1 - ventilation hose fixing assembly, 6 - air outlet assembly, 6.1 - through hole, 6.2 - connecting cylinder, 6.3 - central connecting tube, 6.4 - guide hole, 6.5 - mounting hole, 6.6 - fourth guide column, 6.7 - second elastic element, 6.8 - first oblique wedge, 6.9 - gap, 6.10 - strip-shaped groove, 6.11 - guide groove, 6.12 - positioning protrusion, 6.13 - plate, 6.14 - limit element, 6.14.1 - inclined surface, 6.15 - limit buckle, 7 - protective cover, 7.1 - guide strip, 7.2 - second guide column, 7.3 - anti-slip groove, 7.4 - first elastic element, 7.5 - relief groove, 7.6 - positioning groove, 7.7 - actuating protrusion, 7.8 - protective cover fixing column, 7.9 - guide protrusion, 7.10 - locking positioning groove, 8 - ventilation hose, 9 - limit bracket, 9.1 - rod, 9.2 - second oblique wedge, 9.3 - third guide column, 9.4 - inclined surface, 9.5 - actuator, 10 - mask, 11 - screw.

### DETAILED DESCRIPTION OF THE EMBODIMENTS

Hereinafter, a more detailed explanation of the present invention will be provided through embodiments and in conjunction with the accompanying drawings. The same features/components are labeled with the same reference numerals in all the drawings.

### Embodiment 1

As shown in FIG. 1 to FIG. 17, a sliding protection device for a ventilator air outlet includes a ventilator main body and a ventilation hose 8, the ventilator main body includes an upper cover 1 and a lower cover 2 of the ventilator (the upper cover 1 and the lower cover 2 form a housing), and a space formed by the upper cover 1 and the lower cover 2 is mounted with a fan assembly 3, a control panel 4, a water tank 5, and an air outlet assembly 6. When the ventilator is used, the ventilation hose 8 is inserted into the air outlet assembly 6. After use, the ventilation hose 8 is removed from the air outlet assembly 6. The fan assembly 3 includes a fan air inlet 3.1, a fan body 3.2, and a fan air outlet 3.3. The water tank 5 includes a water tank air inlet 5.1 and a water tank air outlet 5.2. Air in an external environment enters the fan body 3.2 from the fan air inlet 3.1 and is compressed, the compressed air enters the water tank air inlet 5.1 and the water tank air outlet 5.2 sequentially from the fan air outlet 3.3 to adjust humidity, and finally, the air enters the ventilation hose 8 through the air outlet assembly 6. The air outlet assembly 6 serves as the air outlet of the ventilator.

As shown in FIG. 2 to FIG. 8, the air outlet assembly 6 is located inside the upper cover 1 (i.e., below the upper cover 1), the air outlet assembly 6 includes a connecting cylinder 6.2 and a central connecting tube 6.3; one end of the connecting cylinder 6.2 is connected to the upper cover 1, another end of the connecting cylinder 6.2 is connected to a periphery of the central connecting tube 6.3, and there is a gap between the connecting cylinder 6.2 and the central connecting tube 6.3; and an end of the central connecting tube 6.3 near the upper cover 1 does not protrude from the upper cover 1. A lower end of the central connecting tube 6.3 of the air outlet assembly 6 is in communication with the water tank air outlet 5.2. As the air outlet of the ventilator, the air outlet assembly 6 functions to provide a reliable mounting interface for the ventilation hose 8. Therefore, the air outlet assembly 6 needs to have certain rigidity and is preferably made by integral molding.

As shown in FIG. 2 to FIG. 10, the upper cover 1 is provided with a fixing seat 1.1, and a protective cover 7 is slidably provided on the fixing seat 1.1; and the fixing seat 1.1 is fixedly connected to the upper cover 1. The fixing seat 1.1 may be integrally formed with the upper cover 1, or formed as a separate component and then assembled with the upper cover 1. The fixing seat 1.1 has roughly a rectangular structure protruding from the upper cover 1. The fixing seat 1.1 is provided with an opening 1.1.1 corresponding to the air outlet assembly 6. Correspondingly, when the fixing seat 1.1 is integrally formed with the upper cover 1, the upper cover 1 is provided with the opening 1.1.1. When the fixing seat 1.1 and the upper cover 1 are separate components assembled with each other, it can be understood that both the fixing seat 1.1 and the upper cover 1 are provided with the openings 1.1.1, and the openings of the two correspond to each other. Alternatively, only the upper cover 1 is provided with the opening 1.1.1, while the fixing seat 1.1 is arranged on one side of the opening 1.1.1 (not shown in the figure), and the fixing seat 1.1 does not extend in length to cover the opening 1.1.1 of the housing. In this case, no additional opening is required on the fixing seat 1.1. The protective cover 7 has an open state and a closed state. When the protective cover 7 is in the closed state, the protective cover 7 covers (closes) the opening 1.1.1. When the protective cover 7 is in the open state, the air outlet assembly 6 is in an exposed state, and the ventilation hose 8 is capable of passing through the opening 1.1.1 and be connected to the air outlet assembly 6. An outer surface of the protective cover 7 is further provided with an anti-slip groove 7.3. The anti-slip groove 7.3 facilitates user operation on the protective cover 7, thereby causing the protective cover 7 to slide and open. It should be noted that in the above implementation, the fixing seat 1.1 protrudes from the upper cover 1. However, a person skilled in the art can understand that the fixing seat 1.1 may be arranged on an inner surface of the upper cover 1, or even the fixing seat 1.1 may be a plate-like element located inside the upper cover 1, as long as the fixing seat 1.1 provides sliding positioning and guidance for the protective cover 7.

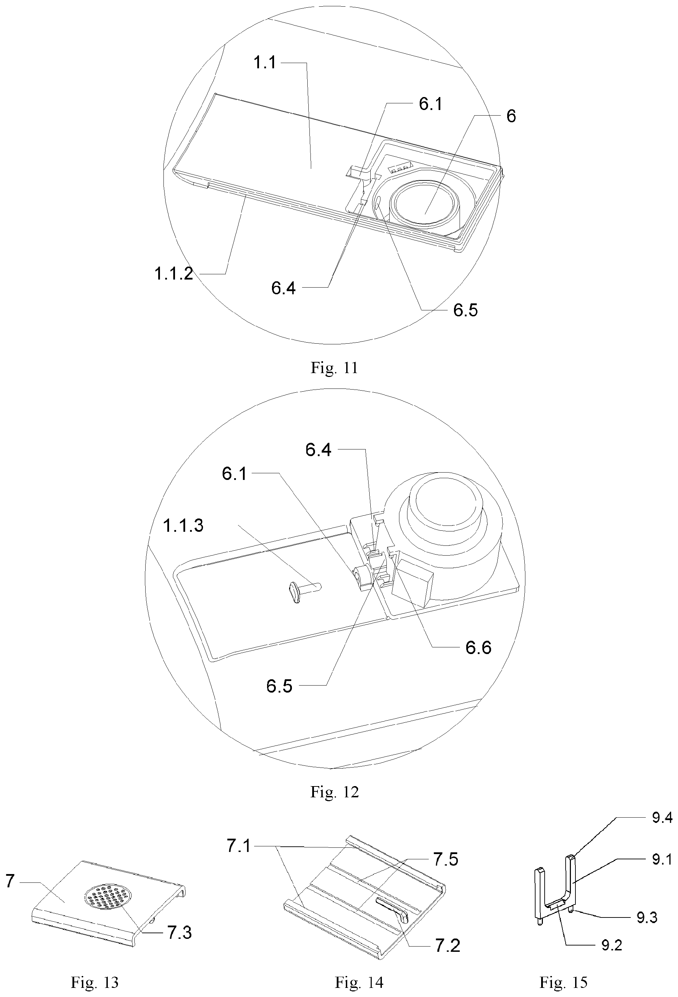

As shown in FIG. 12 to FIG. 14, in order to facilitate the automatic recovery of the protective cover 7 to the closed state, a lower surface of the fixing seat 1.1 is provided with a first guide column 1.1.3, a lower surface of the protective cover 7 is provided with a second guide column 7.2, and two ends of a first elastic element 7.4 (spiral spring) are sleeved on the first guide column 1.13 and the second guide column 7.2 respectively. In order to facilitate the passing of the first elastic element 7.4, a side of the air outlet assembly 6 is provided with a through hole 6.1. The first elastic element 7.4 applies an elastic force to recover the protective cover 7 to the closed state. When the ventilator is not in use, the first elastic element 7.4 ensures that the protective cover 7 is always in the closed state, that is, the protective cover 7 closes the opening 1.1.1.

As shown in FIG. 11 and FIG. 14, both sides of the fixing seat 1.1 are provided with guide grooves 1.1.2, and the protective cover 7 is provided with a guide strip 7.1 that matches the fixing seat 1.1. The guide strip and the guide groove ensure that the protective cover 7 is stably, reliably, and slidably mounted on the slide cover mounting seat 1.1. A person skilled in the art can understand that the guide strip 7.1 may be provided on the fixing seat 1.1, and the guide groove 1.1.2 may be provided on the protective cover 7, as long as the protective cover 7 slides stably relative to the fixing seat 1.1.

In order to automatically recover the protective cover 7 to the closed state, the ventilator of the present invention is further provided with an elastic limiting assembly that automatically responds to the insertion of the ventilation hose 8 into the air outlet assembly 6. The elastic limiting assembly mainly includes a second elastic element 6.7, a limit bracket 9, and a first oblique wedge 6.8;

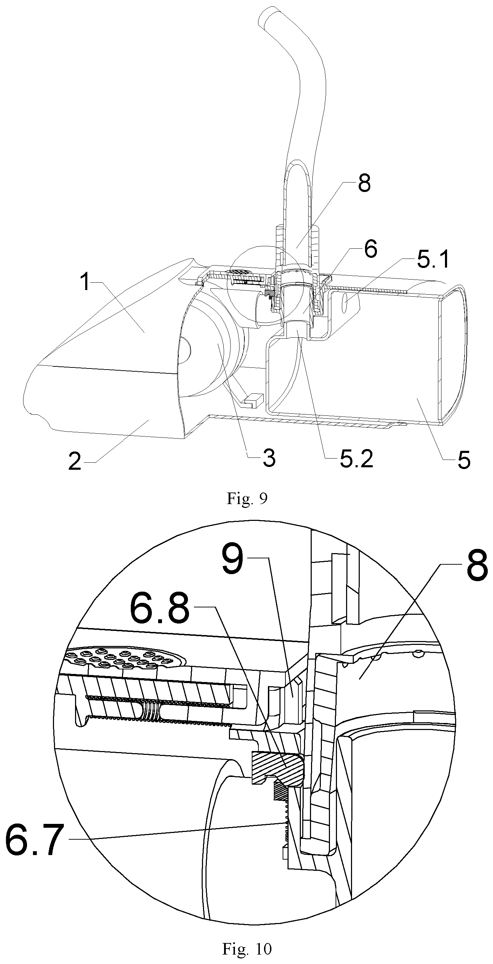

As shown in FIG. 10 to FIG. 17, the limit bracket 9 includes two parallel rods 9.1 and a second oblique wedge 9.2, where the second oblique wedge 9.2 is connected between the two rods 9.1; the connecting cylinder 6.2 is provided with a guide hole 6.4, and the rod 9.1 of the limit bracket 9 extends through the guide hole 6.4, so that the limit bracket 9 is reliably and slidably connected to the air outlet assembly 6; the limit bracket 9 is provided with a third guide column 9.3, the connecting cylinder 6.2 is provided with a fourth guide column 6.6; two ends of a second elastic element 6.7 (spiral spring) are sleeved on the third guide column 9.3 and the fourth guide column 6.6 respectively, and the second elastic element 6.7 is configured to apply an elastic force to move the limit bracket 9 towards the protective cover 7; and an elastic force direction of the first elastic element 7.4 is perpendicular to an elastic force direction of the second elastic element 6.7. The connecting cylinder 6.2 is provided with a through mounting hole 6.5 (a form of channel), and the first oblique wedge 6.8 matching the second oblique wedge 9.2 is provided to extend through the mounting hole 6.5 (generally, a bias spring (not shown in the figure) is required to ensure that the first oblique wedge 6.8 does not fall off from the mounting hole 6.5). When the protective cover 7 is in the open state, the air outlet assembly 6 is in the exposed state, and the protective cover 7 abuts against the limit bracket 9 under the action of the first elastic element 7.4 and is locked (limited). In response to the insertion of the ventilation hose 8 into the gap 6.9 between the connecting cylinder 6.2 and the central connecting tube 6.3, the first oblique wedge 6.8 is slidable in the mounting hole 6.5. By using the oblique wedge, when the ventilation hose 8 is inserted into the gap 6.9, the ventilation hose 8 squeezes the first oblique wedge 6.8 to move horizontally. The movement of the first oblique wedge 6.8 enables the limit bracket 9 to move downward, and the movement of the limit bracket 9 releases the locking of the protective cover 7, so that the protective cover 7 abuts against the ventilation hose 8 under the action of the first elastic element 7.4. When the ventilation hose 8 is removed, the protective cover 7 automatically recovers to the closed state under the action of the first elastic element 7.4. In order to ensure that the first oblique wedge 6.8 moves under the action of the ventilation hose 8, the portion of the first oblique wedge 6.8 that is located within the gap 6.9 is usually a round head or an inclined surface, thereby ensuring the movement of the first oblique wedge 6.8 in response to the insertion of the ventilation hose 8.

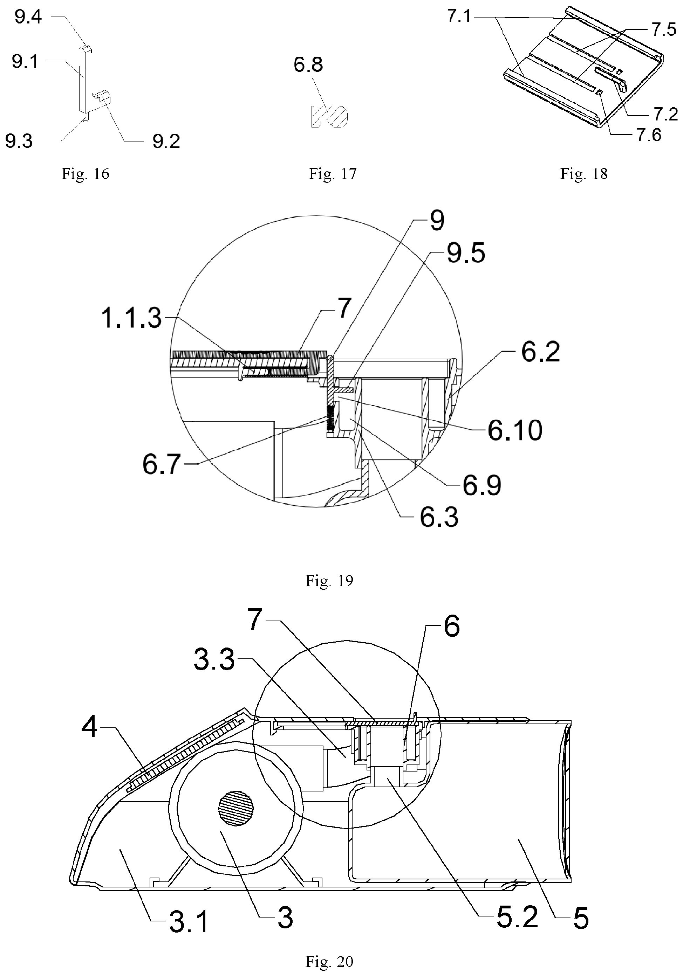

In one implementation, the limit bracket 9 does not necessarily include two parallel rods 9.1, but may include only one rod 9.1, as shown in FIG. 16.

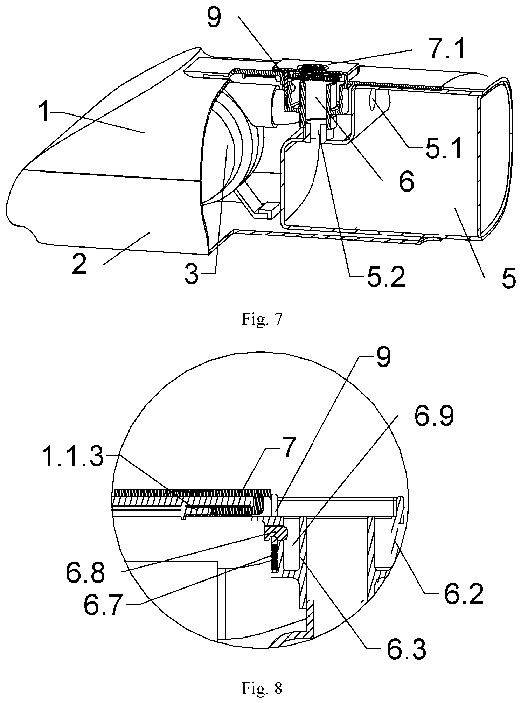

Preferably, as shown in FIG. 14, an inner surface of the protective cover 7 is provided with a relief groove 7.5 corresponding to the limit bracket 9. The relief groove 7.5 minimizes mutual interference between the protective cover 7 and the limit bracket 9 during sliding of the protective cover 7. The relief groove 7.5 is not a through groove, as shown in FIG. 14. The relief groove 7.5 does not extend to an edge of the protective cover 7 in the left-to-right direction. Correspondingly, the limit bracket 9 has an inclined surface 9.4 at an end near the protective cover 7. When the protective cover 7 slides for opening, the limit bracket 9 is located in the relief groove 7.5. The inclined surface 9.4 of the limit bracket 9 helps the limit bracket 9 to disengage from the relief groove 7.5 and ultimately abut against the right edge of the protective cover 7 to achieve locking of the protective cover 7 (as shown in FIG. 8).

In the above implementation, as shown in FIG. 8, the limit bracket 9 abuts against the right edge of the protective cover 7 to lock the protective cover 7 in the open state. In another implementation, as shown in FIG. 18, the inner surface of the protective cover 7 is further provided with a positioning groove 7.6. When the protective cover 7 is in the open state, the limit bracket 9 abuts against a side wall of the positioning groove 7.6. By the positioning groove 7.6, the limit bracket 9 does not necessarily abut against the edge of the protective cover 7, facilitating freer arrangement of the limit bracket 9.

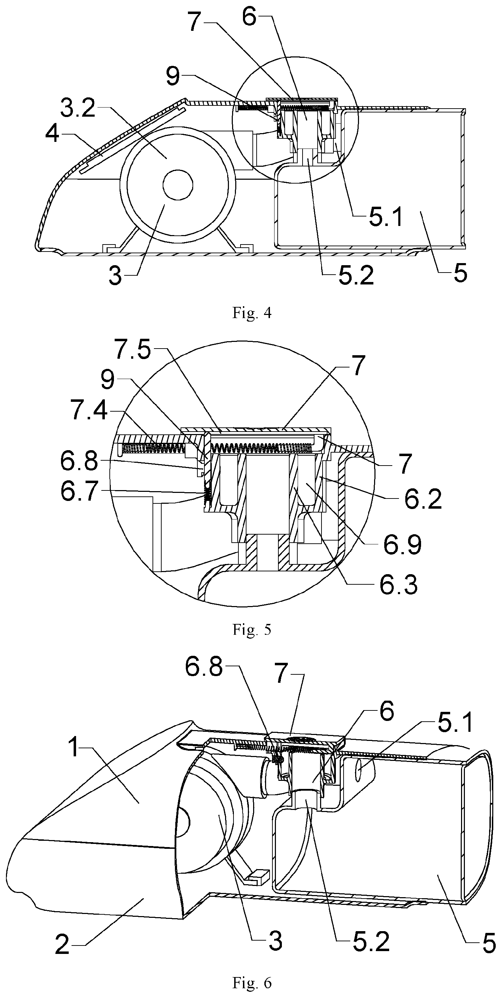

The working principle of the sliding protective cover of the ventilator in the present invention is as follows: When the ventilator is not in operation, the protective cover 7 covers the air outlet assembly 6 under the action of the first elastic element 7.4, as shown in FIG. 3 and FIG. 5. In this state, the protective cover 7 protects the air outlet and internal pipelines of the ventilator from contamination by external contaminants.

The user pushes the anti-slip groove 7.3 on the protective cover 7, so that the protective cover 7 slides backwards, and the air outlet assembly 6 of the ventilator is exposed. The protective cover 7, after being slid to the rear of the limit bracket 9, is pressed and limited by an upper end of the limit bracket 9. After the anti-slip groove 7.3 is released, the protective cover 7 will not rebound, as shown in FIG. 8. At this point, the protective cover 7 is in the open state, the opening 1.1.1 is not covered, and the air outlet assembly 6 is exposed.

The user inserts the ventilation hose 8. During insertion, a front end of the ventilation hose 8 pushes the first oblique wedge 6.8 outward, and the first oblique wedge 6.8 moves backward. Due to the cooperation between the second oblique wedge 9.2 and the first oblique wedge 6.8, the first oblique wedge 6.8 drives the second oblique wedge 9.2 to move downward, so that the limit on the protective cover 7 is released. Because the limit bracket 9 is located below the protective cover 7, the protective cover 7 slides forward under the action of the first elastic element 7.4 to press against the ventilation hose 8, as shown in FIG. 9 and FIG. 10.

When the user stops using the ventilator and removes the ventilation hose 8, the protective cover 7 continues to slide forward under the action of the first elastic element 7.4 until the protective cover completely covers the opening 1.1.1, thereby providing protection for the air outlet assembly 6 of the ventilator. At this point, the protective cover 7 is in the closed state, and the opening 1.1.1 is covered by the protective cover 7, so that the air outlet assembly 6 is not exposed.

In another implementation, as shown in FIG. 19, an actuator 9.5 is provided on the limit bracket 9 of the elastic limiting assembly; the connecting cylinder 6.2 is provided with a strip-shaped groove 6.10 (another form of channel) passing through the wall of the connecting cylinder 6.2, the actuator 9.5 extends through the strip-shaped groove 6.10, and an end of the actuator 9.5 is located in the gap 6.9 between the connecting cylinder 6.2 and the central connecting tube 6.3. The actuator 9.5 is preferably a rod-like element. When the ventilation hose 8 is inserted into the gap 6.9, the ventilation hose 8 pushes the actuator 9.5 to move downward in the strip-shaped groove. The movement of the actuator 9.5 naturally drives the limit bracket 9 to move, and the movement of the limit bracket 9 releases the locking of the protective cover 7, so that the protective cover 7 abuts against the ventilation hose 8 under the action of the first elastic element 7.4, thereby achieving the purpose of releasing the locking of the protective cover 7 by the limit bracket 9 in response to the insertion of the ventilation hose 8 into the air outlet assembly 6. A person skilled in the art can understand that other possible ways can also be used to achieve the technical effect of releasing the locking of the protective cover 7 by the limit bracket 9 in response to the insertion of the ventilation hose 8 into the air outlet assembly 6.

### Embodiment 2

As shown in FIG. 20 to FIG. 37, a ventilator includes a ventilator main body and a ventilation hose 8, the ventilator main body includes an upper cover 1 and a lower cover 2 of the ventilator (the upper cover 1 and the lower cover 2 form a housing), and a space formed by the upper cover 1 and the lower cover 2 is mounted with a fan assembly 3 (an example of a fan), a control panel 4, a water tank 5, and a ventilation hose fixing assembly 6-1. When the ventilator is used, the ventilation hose 8 is inserted into an air outlet assembly 6 of the ventilation hose fixing assembly 6-1. After use, the ventilation hose 8 is removed from the air outlet assembly 6. The fan assembly 3 includes a fan air inlet 3.1, a fan body 3.2, and a fan air outlet 3.3. The water tank 5 includes a water tank air inlet 5.1 and a water tank air outlet 5.2. Air in an external environment enters the fan body 3.2 from the fan air inlet 3.1 and is compressed, the compressed air enters the water tank air inlet 5.1 and the water tank air outlet 5.2 sequentially from the fan air outlet 3.3 to adjust humidity, and finally, the air enters the ventilation hose 8 through the air outlet assembly 6. The air outlet assembly 6 serves as the air outlet of the ventilator.

As shown in FIG. 20 to FIG. 26, the ventilation hose fixing assembly 6-1 is located inside the upper cover 1 (i.e., below the upper cover 1), and the ventilation hose fixing assembly 6-1 includes a plate 6.13 and the air outlet assembly 6, where the air outlet assembly 6 includes a connecting cylinder 6.2 and a central connecting tube 6.3, the plate 6.13 is fixedly connected to a periphery of one end of the connecting cylinder 6.2 near the upper cover 1, a plane where the plate 6.13 is located is perpendicular to an axial direction of the connecting cylinder 6.2, and the plate 6.13 is roughly parallel to a corresponding portion of the upper cover 1; the central connecting tube 6.3 is located inside the connecting cylinder 6.2, the central connecting tube 6.3 is coaxial with the connecting cylinder 6.2, and there is a gap between the connecting cylinder 6.2 and the central connecting tube 6.3 to accommodate the ventilation hose 8; an end of the connecting cylinder 6.2 away from the plate 6.13 is connected to a periphery of the central connecting tube 6.3, and an end of the central connecting tube 6.3 near the upper cover 1 does not protrude from (is not higher than) the plate 6.13; a surface of the plate 6.13 is provided with a limit element 6.14; and the limit element 6.14 is located on a side of the plate 6.13 away from the connecting cylinder 6.2. A lower end of the air outlet assembly 6 is in communication with the water tank air outlet 5.2 to serve as an air outlet of the ventilator. The ventilation hose fixing assembly 6-1 functions to provide a reliable mounting interface for the ventilation hose 8. The air outlet assembly 6 needs to have certain rigidity and is preferably made by integral molding. A person skilled in the art can understand that the air outlet assembly 6 (including the connecting cylinder 6.2 and the central connecting tube 6.3) and the plate 6.13 may be assembled after being manufactured separately. When the user inserts the ventilation hose 8 to the place between the connecting cylinder 6.2 and the central connecting tube 6, the plate 6.13 is subjected to an external force perpendicular to a plate surface, and the plate 6.13 undergoes elastic deformation and depresses. Correspondingly, the limit element 6.14 is displaced with the elastic deformation of the plate 6.13, to release the position limit on the sliding protective cover 7, thereby achieving automatic closing of the protective cover 7 (details will be described below). The plate 6.13 is fixedly connected to a periphery of one end of the connecting cylinder 6.2 near the upper cover 1, which does not necessarily mean that the plate 6.13 should be arranged around the entire connecting cylinder 6.2. In some optional solutions, the plate 6.13 may extend outward from a portion of the periphery of the connecting cylinder 6.2. It should be noted that the plate 6.13 is an example of a load-bearing element. A person skilled in the art can use other forms of load-bearing elements as long as they undergo elastic deformation when pressed. It should be noted that in the above implementation, the limit element 6.14 is a protruding limit element. A person skilled in the art can understand that the limit element 6.14 may be a limit groove that matches a corresponding limit protrusion on the protective cover 7.

In order to simplify the mounting of the ventilation hose fixing assembly 6-1, the upper cover 1 is provided with a hook 1.2 and a screw post 1.3. one end of the plate 6.13 is engaged with the hook 1.2, and another end of the plate 6.13 is fixed to the screw post 1.3 of the upper cover 1 through a screw 11 (an example of a fastener), that is, one end of the connecting cylinder 6.2 is connected to the upper cover 1 through the plate 6.13. In this way, the ventilation hose fixing assembly 6-1 is quickly fixed to the upper cover 1, and the ventilation hose fixing assembly 6-1 is accurately positioned on the upper cover 1. The end of the plate 6.13 that is engaged with the hook 1.2 is equivalent to a free end. When the plate 6.13 is subjected to a pressure perpendicular to the plane where the plate 6.13 is located, the end of the plate 6.13 that is engaged with the hook 1.2 is capable of slight displacement, making the plate 6.13 more prone to elastic deformation. A person skilled in the art can be aware of various possibilities for the connection between the air outlet assembly 6 and the ventilation hose 8, such as tight fit between the ventilation hose 8 and the inner wall of the connecting cylinder 6.2 (the central connecting tube 6.3 may have a hollow structure to reduce weight), or tight fit between the ventilation hose 8 and the outer wall of the central connecting tube 6 (the connecting cylinder 6.2 may have a hollow structure to reduce weight), or the ventilation hose 8 is in tight fit with both the outer wall of the central connecting tube 6.3 and the inner wall of the connecting cylinder 6.2, so as to transfer air from the air outlet assembly 6 to the ventilation hose 8 without leakage.

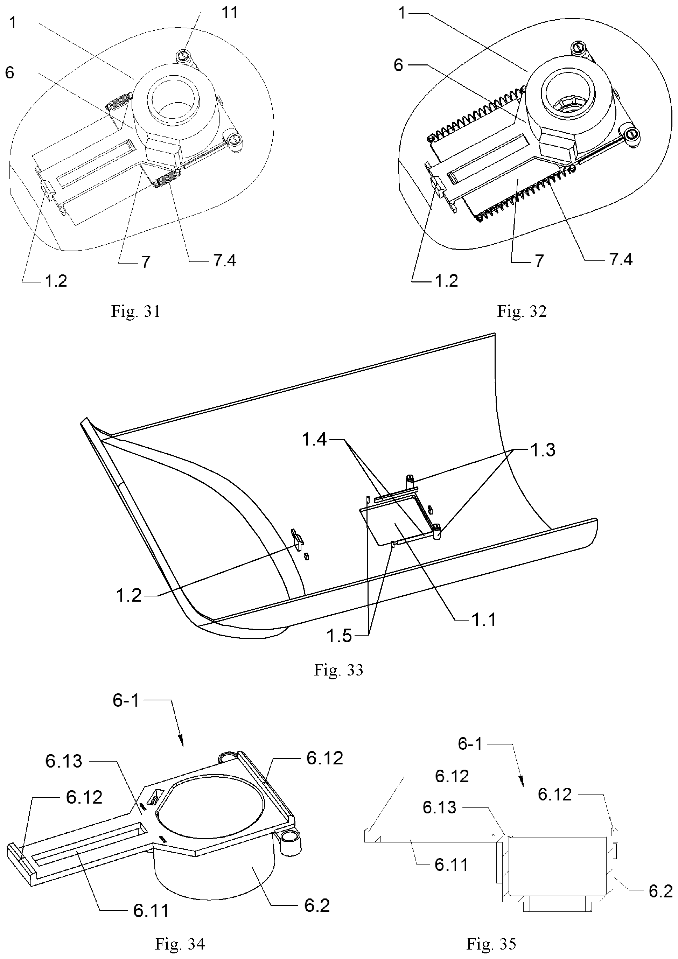

The air outlet assembly 6 may have various forms, for example, the central connecting tube 6.3 is omitted, as shown in FIG. 34 and FIG. 35. At this point, when the ventilation hose 8 is connected to the air outlet assembly 6, the ventilation hose 8 is in tight fit with the inner wall of the connecting cylinder 6.2.

As shown in FIG. 20 to FIG. 24, the upper cover 1 is provided with an opening 1.1.1, and the connecting cylinder 6.2 and the central connecting tube 6.3 of the air outlet assembly 6 correspond to the opening 1.1.1; a protective cover 7 is slidably provided between the upper cover 1 and the plate 6.13, and the protective cover 7 is switchable between a first position (closed state) and a second position (open state); when the protective cover 7 is in the closed state, the protective cover 7 closes (covers) the opening 1.1.1; when the protective cover 7 is in the open state, the air outlet assembly 6 is exposed, and the ventilation hose 8 is capable of passing through the opening 1.1.1 and be connected to the air outlet assembly 6. An outer surface of the protective cover 7 is further provided with an actuating protrusion 7.7. The actuating protrusion 7.7 facilitates user operation on the protective cover 7, thereby causing the protective cover 7 to slide and open.

The protective cover 7 is located between the plate 6.13 and the upper cover 1, and a space for accommodating the protective cover 7 is formed between the plate 6.13 and the upper cover 1, to limit the sliding range of the protective cover 7. Preferably, both ends of the plate 6.13 are provided with positioning protrusions 6.12, the positioning protrusions 6.12 extend from the plate 6.13 towards the upper cover 1, that is, the positioning protrusions 6.12 and the limit element 6.14 are located on the same side surface of the plate 6.13. When the ventilation hose fixing assembly 6-1 is fixed to the upper cover 1, an extension height of the positioning protrusion 6.12 towards the upper cover 1 is greater than a thickness of the protective cover 7, thereby ensuring sliding of the protective cover 7 in the gap between the upper cover 1 and the plate 6.13. In order to facilitate the automatic recovery of the protective cover 7 to the closed state, one end of a first elastic element 7.4 (which may be a tension spring) is connected to the protective cover 7, and another end is connected to the upper cover 1. Specifically, a side of the upper cover 1 is provided with an upper cover fixing column 1.5, the upper cover fixing column 1.5 is provided with a groove (not shown in the figure), and one end of the first elastic element 7.4 is mounted at a corresponding position of the groove; and both sides of the protective cover 7 are provided with protective cover fixing columns 7.8, the protective cover fixing column 7.8 is provided with a groove (not shown in the figure), and another end of the first elastic element 7.4 is mounted at a corresponding position of the groove. A person skilled in the art can understand that another end of the first elastic element 7.4 may be connected to the plate 6.13 of the ventilation hose fixing assembly 6-1. In some cases, the first elastic element 7.4 may be replaced with a compression spring. The first elastic element 7.4 applies an elastic force to recover the protective cover 7 to the closed state. When the ventilator is not in use, the elastic element ensures that the protective cover 7 is always in the closed state, that is, the protective cover 7 closes the opening 1.1.1 of the upper cover 1.

The plate 6.13 and/or the upper cover 1 are provided with guide elements for guiding the sliding of the protective cover 7. The guide elements ensure that the protective cover 7 slides in a predetermined direction, and prevent the protective cover 7 from shaking, falling, etc. Specifically, the guide element includes a guide strip 1.4 arranged on the upper cover 1. A person skilled in the art can understand that the same effect is achieved when the guide strip 1.4 is arranged on the plate 6.13. In order to further improve the stability of sliding guidance, the protective cover 7 is provided with a guide protrusion 7.9, and the plate 6.13 is provided with a guide groove 6.11 correspondingly matching the guide protrusion 7.9. The guide groove 6.11 not only provides sliding guidance for the protective cover 7, but also is more conducive to increasing the elasticity of the plate 6.13, making the plate 6.13 more prone to deformation along the axial direction of the connecting cylinder 6.2.

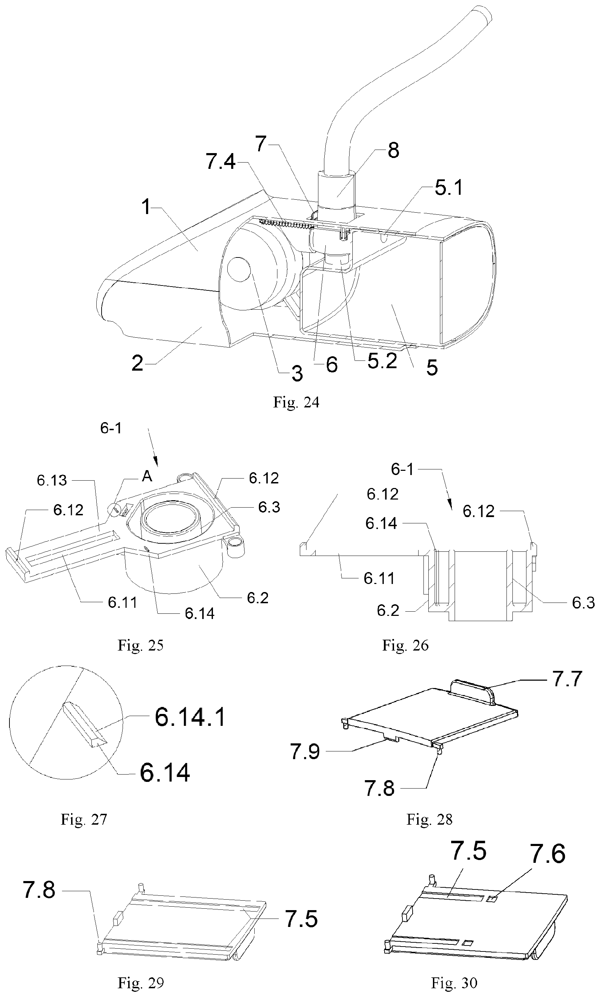

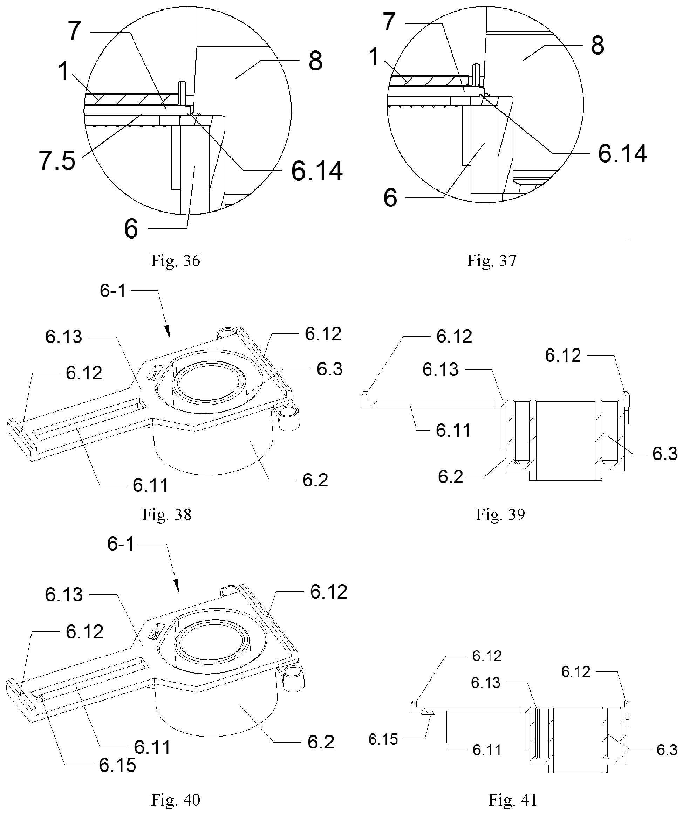

Preferably, as shown in FIG. 28 and FIG. 29, an inner surface of the protective cover 7 is provided with a relief groove 7.5 corresponding to the limit element 6.14. The relief groove 7.5 minimizes mutual interference between the protective cover 7 and the limit element 6.14 during sliding of the protective cover 7. The relief groove 7.5 is not a through groove, as shown in FIG. 29. The relief groove 7.5 does not extend to an edge of the protective cover 7 in the left-to-right direction. Correspondingly, the limit element 6.14 has an inclined surface 6.14.1 on a side near the connecting cylinder 6.2. When the protective cover 7 slides for opening, the limit element 6.14 is located in the relief groove 7.5. The inclined surface 6.14.1 of the limit element 6.14 helps the limit element 6.14 to disengage from the relief groove 7.5 and ultimately abut against the right edge of the protective cover 7 to achieve positioning of the protective cover 7 (as shown in FIG. 27 and FIG. 36). Alternatively, an end side wall of the relief groove 7.5 may be provided with a corresponding inclined surface (as shown in FIG. 36). As shown in FIG. 25 and FIG. 29, two limit elements 6.14 and two parallel relief grooves 7.5 are designed to improve stability.

In the above implementation, the limit element 6.14 abuts against the right edge of the protective cover 7 to position the protective cover 7 in the open state. In another implementation, as shown in FIG. 30, the inner surface of the protective cover is further provided with a positioning groove 7.6. When the protective cover 7 is in the open state, the limit element 6.14 abuts against a side wall of the positioning groove 7.6. By designing the positioning groove 7.6, the limit element 6.14 does not necessarily abut against the edge of the protective cover 7, facilitating freer arrangement of the limit element 6.14, especially facilitating the arrangement of the limit element 6.14 at a greater deformation position of the plate 6.13, such as near a middle position between an end of the plate 6.13 and the connecting cylinder 6.2.

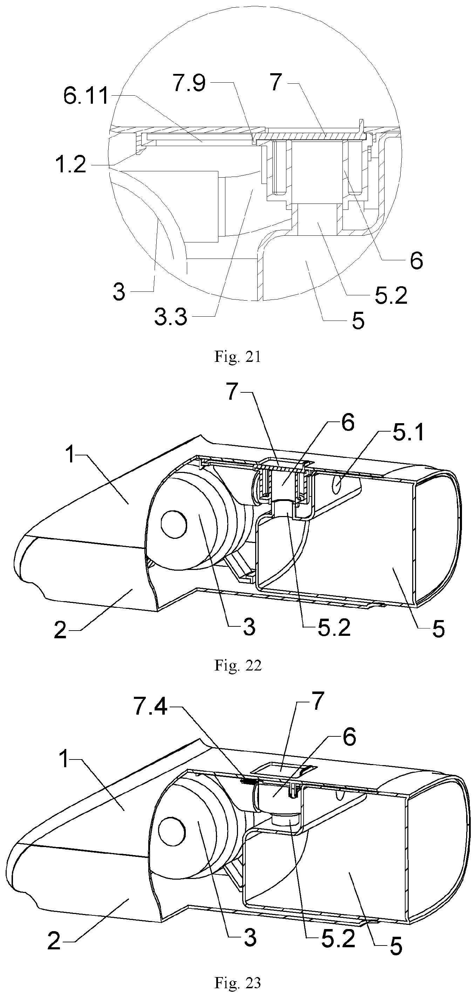

The working principle of the sliding protective cover of the ventilator in the present invention is as follows: When the ventilator is not in operation, the protective cover 7 closes the opening 1.1.1 of the ventilator air outlet under the action of the first elastic element 7.4, as shown in FIG. 21. In this state, the protective cover 7 protects the ventilator air outlet and internal water tank and other pipelines of the ventilator from contamination by external dust and bacteria. When the ventilator is in operation, the user pushes the actuating protrusion 7.7 backward to cause sliding of the protective cover 7 to open (expose) the opening 1.1.1 of the ventilator, the protective cover 7 slides to the left of the limit element 6.14 (or the limit element 6.14 is located in the positioning groove 7.6 of the protective cover 7), and the protective cover 7 abuts against the limit element 6.14 under the action of the first elastic element 7.4 and is limited from rebounding, as shown in FIG. 36. At this point, the protective cover 7 is in the second position (open state).

When the user inserts the ventilation hose 8, due to the tight fit between the ventilation hose 8 and the air outlet assembly 6, the plate 6.13 will be subjected to a pressure perpendicular to the plate 6.13 and directed toward the interior of the ventilator. Under the pressure, the plate 6.13 is elastically deformed inward. The deformation causes the limit element 6.14 to move downward and releases the limit on the protective cover 7. The limit element 6.14 releases the limit on the protective cover 7, and the protective cover 7 will slide forward under the action of the first elastic element 7.4 to press against the ventilation hose 8, as shown in FIG. 37. That is, in response to the insertion operation of connecting the ventilation hose 8 to the air outlet assembly 6, the protective cover 7 presses against the ventilation hose 8.

When the user stops using the ventilator and pulls out the ventilation hose 8, the protective cover 7 continues to slide automatically under the action of the first elastic element 7.4 to a position where the opening 1.1.1 of the ventilator air outlet is closed. At this point, the protective cover 7 is in the first position (closed state).

When the user stops using the ventilator and pulls out the ventilation hose 8, the protective cover 7 continues to slide forward automatically under the action of the first elastic element 7.4 to the position where the opening 1.1.1 is closed. The automatic reset of the protective cover 7 helps prevent the user from forgetting to close the protective cover due to original operating habits after using the ventilator, thereby improving the protective effect. At the same point, the protective cover 7 is capable of automatically resetting without requiring secondary contact by user's hands, thereby further reducing the possibility of contamination of the ventilator air outlet.

### Embodiment 3

As shown in FIGs. 20-24, 28, 31-33, and 38-39, Embodiment 3 differs from Embodiment 2 in that the components such as the limit element 6.14, the relief groove 7.5, and the positioning groove 7.6 in Embodiment 2 are cancelled in Embodiment 3. The components identical to those in Embodiment 2 have the same functions and effects in Embodiment 3, and will not be repeated here. Compared to Embodiment 2, in the solution of Embodiment 3, the protective cover 7 will not abut against the ventilation hose 8 in response to the insertion operation of connecting the ventilation hose 8 to the air outlet assembly 6. By using the technical solution of Embodiment 3, when the ventilator is in operation, the user pushes the actuating protrusion 7.7 backward to cause sliding of the protective cover 7 to open (expose) the opening 1.1.1 of the ventilator. The user presses the protective cover 7 with one hand to prevent it from rebounding, and installs the ventilation hose 8 with the other hand. After the installation of the ventilation hose 8 is completed, the protective cover 7 is released. The protective cover 7 will rebound under the action of the first elastic element 7.4 to press against the ventilation hose 8. The working state is as shown in FIG. 24. When the user stops using the ventilator and pulls out the ventilation hose 8, the protective cover 7 continues to slide forward automatically under the action of the first elastic element 7.4 to the position where the opening 1.1.1 is closed. The automatic reset of the protective cover 7 helps prevent the user from forgetting to close the protective cover due to original operating habits after using the ventilator, thereby improving the protective effect. At the same point, the protective cover 7 is capable of automatically resetting without requiring secondary contact by user's hands, thereby further reducing the possibility of contamination of the ventilator air outlet.

### Variation of Embodiment 3

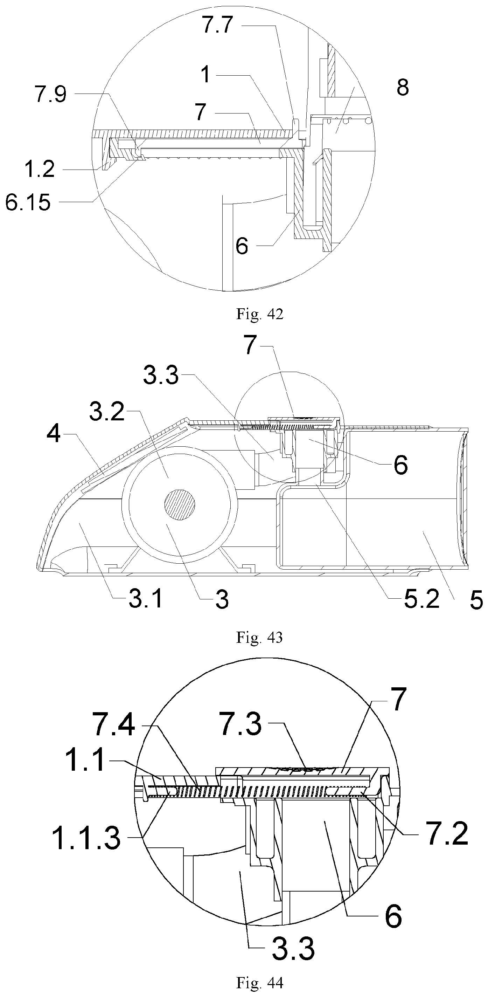

As shown in FIG. 40 to FIG. 42, on the basis of Embodiment 3, a limit buckle 6.15 is further added in the variation. The protective cover 7 is provided with a guide protrusion 7.9, and the plate 6.5 is provided with a guide groove 6.3 correspondingly matching the guide protrusion 7.9; the plate 6.5 is further provided with the limit buckle 6.15 used with the guide protrusion 7.9, and the limit buckle 6.15 is located at an end of the guide groove 6.11 away from the connecting cylinder 6.2. When the protective cover 7 is slid from the closed state to the open state, the guide protrusion 7.9 slides within the guide groove 6.11 until the guide protrusion 7.9 is engaged in the limit buckle 6.15. The limit buckle 6.15 is capable of limiting the protective cover 7 and preventing the protective cover 7 from automatically rebounding. A person skilled in the art can understand that the engaging force between the guide protrusion 7.9 and the limit buckle 6.15 is capable of preventing the protective cover 7 from rebounding. However, when the user applies an external force to the protective cover 7, the guide protrusion 7.9 is capable of disengaging from the limit buckle 6.15, and then the protective cover 7 automatically slides under the action of the elastic element to the position where the opening 1.1.1 of the upper cover 1 is closed.

### Embodiment 4

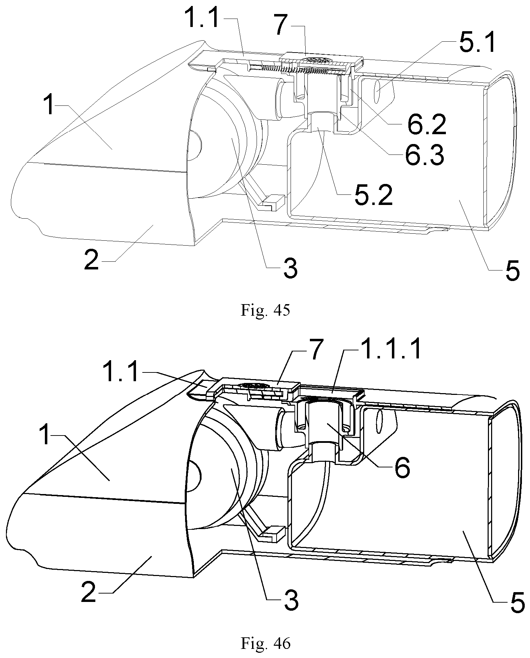

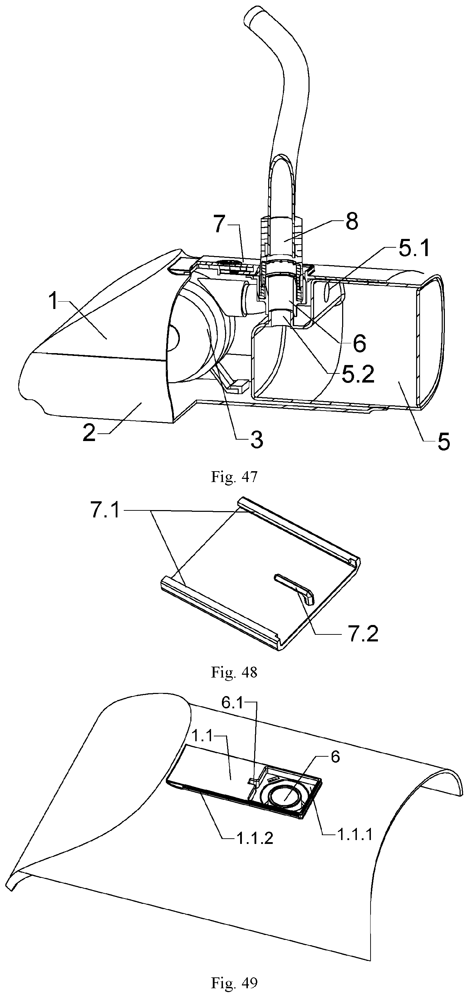

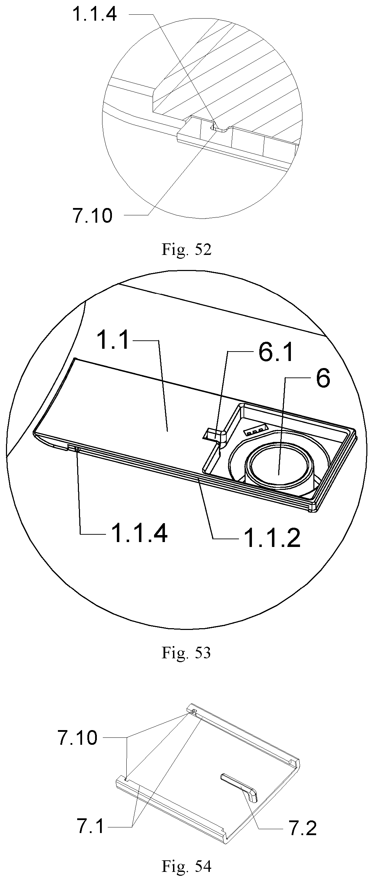

As shown in FIG. 7 and FIGs. 43-54, Embodiment 4 differs from Embodiment 1 in that the components such as the elastic limiting assembly (the second elastic element 6.7, the limit bracket 9, and the first oblique wedge 6.8) in Embodiment 1 are cancelled in Embodiment 4. The components identical to those in Embodiment 1 have the same functions and effects in Embodiment 4, and will not be repeated here. Compared to Embodiment 1, in the solution of Embodiment 4, the protective cover 7 will not abut against the ventilation hose 8 in response to the insertion operation of connecting the ventilation hose 8 to the air outlet assembly 6. By using the technical solution of Embodiment 3, when the ventilator is in operation, the user pushes the anti-slip groove 7.3 backward to cause sliding of the protective cover 7 to open (expose) the opening 1.1.1 of the ventilator. The user presses the protective cover 7 with one hand to prevent it from rebounding, and installs the ventilation hose 8 with the other hand. After the installation of the ventilation hose 8 is completed, the protective cover 7 is released. The protective cover 7 will rebound under the action of the first elastic element 7.4 to press against the ventilation hose 8. The working state is as shown in FIG. 47. When the user stops using the ventilator and pulls out the ventilation hose 8, the protective cover 7 continues to slide forward automatically under the action of the first elastic element 7.4 to the position where the opening 1.1.1 is closed, as shown in FIG. 45. The automatic reset of the protective cover 7 helps prevent the user from forgetting to close the protective cover due to original operating habits after using the ventilator, thereby improving the protective effect. At the same point, the protective cover 7 is capable of automatically resetting without requiring secondary contact by user's hands, thereby further reducing the possibility of contamination of the ventilator air outlet.

### Variation of Embodiment 4

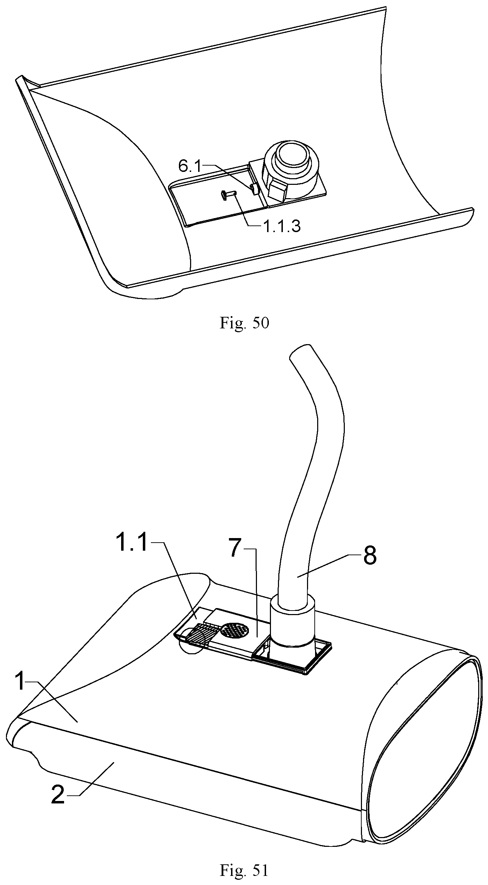

As shown in FIG. 51 to FIG. 54, on the basis of Embodiment 4, a locking positioning protrusion 1.1.4 and a locking positioning groove 7.10 are further added in the variation. The fixing seat 1.1 is provided with the locking positioning protrusion 1.1.4, and the protective cover 7 is provided with the locking positioning groove 7.10 that matches the locking positioning protrusion 1.1.4. When the protective cover 7 slides from the closed state to the open state, the protective cover 7 slides on the fixing seat 1.1 until the locking positioning protrusion 1.1.4 is engaged in the locking positioning groove 7.10. The locking positioning groove 7.10 is capable of limiting the protective cover 7 and preventing the protective cover 7 from automatically rebounding. A person skilled in the art can understand that the engaging force between the locking positioning protrusion 1.1.4 and the locking positioning groove 7.10 is capable of preventing the protective cover 7 from rebounding. However, when the user applies an external force to the protective cover 7, the locking positioning protrusion 1.1.4 is capable of disengaging from the locking positioning groove 7.10, and then the protective cover 7 automatically slides under the action of the elastic element to the position where the opening 1.1.1 of the upper cover 1 is covered. It should be noted that the locking positioning protrusions 1.1.4 shown in FIG. 51 to FIG. 54 are located on both sides of the fixing seat 1.1, but the locking positioning protrusion 1.1.4 may alternatively be arranged in other positions, such as on the upper surface of the fixing seat 1.1. Correspondingly, the locking positioning groove 7.10 may be arranged on the lower surface of the protective cover 7.

The embodiments of the present invention are described above with reference to the accompanying drawings. The embodiments of the present invention and the features of the embodiments may be combined with each other on a non-conflict basis. The present invention is not limited to the foregoing specific implementations, and the foregoing specific implementations are merely illustrative but not restrictive. Many forms may also be made by those of ordinary skill in the art under the enlightenment of the present invention without departing from the purpose of the present invention and the scope of the claims, and these forms fall into the scope of the present invention.

## Claims

1. A sliding protection device for a ventilator air outlet, comprising a ventilator main body, a ventilation hose (8), and a protective cover (7), wherein the ventilator main body comprises a housing, a fan assembly (3), and an air outlet assembly (6); the fan assembly (3) is provided with a fan air inlet (3.1) and a fan air outlet (3.3), the air outlet assembly (6) is in communication with the fan air outlet (3.3), and the air outlet assembly (6) is configured to be connected to the ventilation hose (8), wherein
the air outlet assembly (6) is located in the housing, and the housing is provided with an opening (1.1.1) corresponding to the air outlet assembly (6); the protective cover (7) is arranged in a manner of sliding relative to the housing, and the protective cover (7) is capable of being opened and closed; when the protective cover (7) is in a closed state, the protective cover (7) covers the air outlet assembly (6); when the protective cover (7) is in an open state, the air outlet assembly (6) is in an exposed state.

2. The sliding protection device for the ventilator air outlet according to claim 1, wherein the air outlet assembly (6) comprises a connecting cylinder (6.2) and a central connecting tube (6.3), one end of the connecting cylinder (6.2) is connected to the housing, another end of the connecting cylinder (6.2) is connected to a periphery of the central connecting tube (6.3); there is a gap between the connecting cylinder (6.2) and the central connecting tube (6.3), and an end of the central connecting tube (6.3) near the housing does not protrude from the housing.

3. The sliding protection device for the ventilator air outlet according to claim 1 or 2, wherein in response to an insertion operation of connecting the ventilation hose (8) to the air outlet assembly (6), the protective cover (7) abuts against the ventilation hose (8).

4. The sliding protection device for the ventilator air outlet according to claim 3, further comprising a first elastic element (7.4) and an elastic limiting assembly, wherein when the protective cover (7) is opened, the elastic limiting assembly moves upward to lock the protective cover (7); and
when the ventilation hose (8) is inserted into the air outlet assembly (6), the elastic limiting assembly is driven to move downward and release the locking of the protective cover (7); when the ventilation hose (8) is removed from the air outlet assembly (6), the protective cover (7) is closed under the action of the first elastic element (7.4).

5. The sliding protection device for the ventilator air outlet according to claim 4, wherein a fixing seat (1.1) is provided on the housing, and the protective cover (7) is slidably provided on the fixing seat (1.1); one end of the first elastic element (7.4) is connected to the protective cover (7), and another end of the first elastic element (7.4) is connected to the housing or the fixing seat (1.1).

6. The sliding protection device for the ventilator air outlet according to claim 4, wherein the elastic limiting assembly comprises a second elastic element (6.7) and a limit bracket (9), wherein the second elastic element (6.7) is configured to apply an elastic force to move the limit bracket (9) towards the protective cover (7).

7. The sliding protection device for the ventilator air outlet according to claim 6, wherein the limit bracket (9) is slidably arranged on the air outlet assembly (6); one end of the second elastic element (6.7) is connected to the limit bracket (9), another end of the second elastic element (6.7) is connected to the air outlet assembly (6), and an elastic force direction of the second elastic element (6.7) is perpendicular to that of the first elastic element (7.4).

8. The sliding protection device for the ventilator air outlet according to claim 7, wherein the connecting cylinder (6.2) is provided with a guide hole (6.4), and the limit bracket (9) extends through the guide hole (6.4).

9. The sliding protection device for the ventilator air outlet according to claim 7 or 8, wherein a channel is provided on a wall of the connecting cylinder (6.2), a movable actuator (9.5) extends through the channel, and an end of the actuator (9.5) is located in the gap between the connecting cylinder (6.2) and the central connecting tube (6.3).

10. The sliding protection device for the ventilator air outlet according to claim 9, wherein the actuator is a first oblique wedge (6.8), and a second oblique wedge (9.2) matching the first oblique wedge (6.8) is provided on the limit bracket (9); when the ventilation hose (8) is inserted into the gap between the connecting cylinder (6.2) and the central connecting tube (6.3), the ventilation hose (8) actuates the first oblique wedge (6.8) to slide horizontally in the channel, so that the second oblique wedge (9.2) is driven to move the limit bracket (9) downward.

11. The sliding protection device for the ventilator air outlet according to claim 10, wherein the limit bracket (9) comprises two parallel rods (9.1), and the second oblique wedge (9.2) is connected between the two rods (9.1).

12. The sliding protection device for the ventilator air outlet according to claim 9, wherein the channel is a strip-shaped groove (6.10) extending vertically and passing through the wall of the connecting cylinder (6.2), and the actuator is a cross bar (9.5) that is arranged on the limit bracket (9) and is movable up and down along the strip-shaped groove (6.10); when the ventilation hose (8) is inserted into the gap between the connecting cylinder (6.2) and the central connecting tube (6.3), the cross bar (9.5) is pressed down and drives the limit bracket (9) to move downward.

13. The sliding protection device for the ventilator air outlet according to claim 6, wherein an inner surface of the protective cover (7) is provided with a relief groove (7.5) corresponding to the limit bracket (9).

14. The sliding protection device for the ventilator air outlet according to claim 6, wherein the inner surface of the protective cover (7) is further provided with a positioning groove (7.6), and when the protective cover (7) is in the open state, the limit bracket (9) abuts against a side wall of the positioning groove (7.6).

15. The sliding protection device for the ventilator air outlet according to claim 3, wherein the ventilator main body further comprises a first elastic element (7.4) and a plate (6.13) that is located in the housing, and the air outlet assembly (6) is connected to the plate (6.13); the protective cover (7) is slidably provided between the housing and the plate (6.13); a limit element (6.14) is provided on the plate (6.13), and the protective cover (7) is limited by the limit element (6.14) when opened;
when the ventilation hose (8) is connected to the air outlet assembly (6), the plate (6.13) is pressed down and undergoes elastic deformation, so that the limit element (6.14) releases the limit on the protective cover (7); and
after the ventilation hose (8) is disengaged from the air outlet assembly (6), the protective cover (7) is closed under the action of the first elastic element to cover the air outlet assembly (6).

16. The sliding protection device for the ventilator air outlet according to claim 15, wherein one end of the first elastic element is connected to the protective cover (7), and another end of the first elastic element is connected to the housing or the plate (6.13).

17. The sliding protection device for the ventilator air outlet according to claim 15, wherein the plate (6.13) is fixedly connected to a periphery of one end of the connecting cylinder (6.2) near the housing, and a plane where the plate (6.13) is located is perpendicular to an axial direction of the connecting cylinder (6.2); a surface of the plate (6.13) is provided with the limit element (6.14), and the limit element (6.14) is located on a side of the plate (6.13) away from the connecting cylinder (6.2).

18. The sliding protection device for the ventilator air outlet according to claim 16 or 17, wherein the limit element (6.14) has an inclined surface (6.14.1) on a side near the connecting cylinder (6.2).

19. The sliding protection device for the ventilator air outlet according to claim 15, wherein a hook (1.2) is provided on the housing; one end of the plate (6.13) is engaged with the hook (1.2), and another end of the plate (6.13) is fixed to the housing by a fastener.

20. The sliding protection device for the ventilator air outlet according to claim 1 or 2, wherein the ventilator main body further comprises a first elastic element (7.4) and a plate (6.13) located in the housing; the protective cover (7) is slidably provided between the housing and the plate (6.13); one end of the first elastic element (7.4) is connected to the protective cover (7), and another end of the first elastic element (7.4) is connected to the housing or the plate (6.13); and the first elastic element (7.4) is configured to apply an elastic force to switch the protective cover (7) from the open state to the closed state.

21. The sliding protection device for the ventilator air outlet according to claim 20, wherein the plate (6.13) is fixedly connected to a periphery of one end of the connecting cylinder (6.2) near the housing, and a plane where the plate (6.13) is located is perpendicular to an axial direction of the connecting cylinder (6.2); both ends of the plate (6.13) are provided with positioning protrusions (6.12), and the positioning protrusions (6.12) extend from the plate (6.13) towards the housing.

22. The sliding protection device for the ventilator air outlet according to claim 20, wherein a hook (1.2) is provided on the housing; one end of the plate (6.13) is engaged with the hook (1.2), and another end of the plate (6.13) is fixed to the housing by a fastener.

23. The sliding protection device for the ventilator air outlet according to claim 20, wherein the protective cover (7) is provided with a guide protrusion (7.9), and the plate (6.13) is provided with a guide groove (6.11) correspondingly matching the guide protrusion (7.9); the plate (6.13) is further provided with a limit buckle (6.15) used with the guide protrusion (7.9), and the limit buckle (6.15) is located at an end of the guide groove (6.11) away from the connecting cylinder (6.2).

24. The sliding protection device for the ventilator air outlet according to claim 1 or 2, wherein a fixing seat (1.1) is provided on the housing, and the protective cover (7) is slidably provided on the fixing seat (1.1); the sliding protection device further comprises a first elastic element (7.4); one end of the first elastic element (7.4) is connected to the protective cover (7), and another end of the first elastic element (7.4) is connected to the housing or the fixing seat (1.1).

25. The sliding protection device for the ventilator air outlet according to claim 24, wherein the fixing seat (1.1) is provided with a guide strip (7.1) and/or a guide groove (1.1.2), and the protective cover (7) is provided with a guide groove (1.1.2) and/or a guide strip (7.1) matching the fixing seat (1.1).

26. The sliding protection device for the ventilator air outlet according to claim 24, wherein the fixing seat (1.1) is provided with a locking positioning protrusion (1.1.4), and the protective cover (7) is provided with a locking positioning groove (7.10) matching the locking positioning protrusion (1.1.4).
